(19)

**Europäisches Patentamt**
**European Patent Office**
**Office européen des brevets**

(11) **EP 4 692 129 A1**

(12) **EUROPEAN PATENT APPLICATION**
published in accordance with Art. 153(4) EPC

(43) Date of publication:
**11.02.2026 Bulletin 2026/07**

(21) Application number: **24779749.1**

(22) Date of filing: **19.03.2024**

(51) International Patent Classification (IPC):
*C08B 37/00* (2006.01)     *A23L 5/00* (2016.01)
*A23L 29/00* (2016.01)     *A23L 33/105* (2016.01)
*A23L 33/125* (2016.01)     *A61K 31/715* (2006.01)
*A61K 36/48* (2006.01)     *A61P 11/10* (2006.01)
*A61P 11/14* (2006.01)     *A61P 37/04* (2006.01)
*C08B 37/08* (2006.01)

(52) Cooperative Patent Classification (CPC):
**C08B 37/003; A23L 5/00; A23L 29/00;**
**A23L 29/238; A23L 33/105; A23L 33/125;**
**A61K 31/715; A61K 36/48; A61P 1/00;**
**A61P 11/10; A61P 11/14; A61P 37/02; A61P 37/04;**
**C08B 37/006**

(86) International application number:
**PCT/JP2024/010763**

(87) International publication number:
**WO 2024/203624 (03.10.2024 Gazette 2024/40)**

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**
Designated Extension States:
**BA**
Designated Validation States:
**GE KH MA MD TN**

(30) Priority: **31.03.2023 JP 2023058589**

(71) Applicant: **Nagayoshi Pharmaceutical Co., Ltd.**
**Tokyo 101-0051 (JP)**

(72) Inventors:
• **WANG,Xiangtao**
  **Beijing 100193 (CN)**

• **LIU,Xinxin**
  **Beijing 100193 (CN)**
• **ARIGA,Yosuke**
  **Urayasu-shi, Chiba 279-0004 (JP)**
• **JAMES,Wei**
  **Urayasu-shi, Chiba 279-0004 (JP)**
• **HAN,Meihua**
  **Beijing 100193 (CN)**
• **HAN,Jianwei**
  **Beijing 100193 (CN)**

(74) Representative: **Schön, Christoph**
  **Dr. Schön, Neymeyr & Partner mbB**
  **Bavariaring 26**
  **80336 München (DE)**

(54) **CAROB POLYSACCHARIDE CAPABLE OF SUPPRESSING COUGH, REDUCING PHLEGM AND ENHANCING IMMUNITY, PREPARATION METHOD THEREFOR, AND USE THEREOF**

(57)     The carob polysaccharide comprises two types of monosaccharides, galactose and glucose, wherein the molar ratio of galactose to glucose is (88-92):(0.8-1.2), and the carob polysaccharide is linked and combined through glycosidic bonds of $(1\rightarrow4)$-Glu, $(1\rightarrow3,4,6)$-Glu, $(1\rightarrow3)$-Gal, $(3\rightarrow6)$-Gal, $(2\rightarrow4,6)$-Gal, $(2\rightarrow6)$-Gal, and $(1\rightarrow)$-Gal. The carob polysaccharide can improve the respiratory tract, stop coughing, reduce phlegm, and also enhance immunity.

**EP 4 692 129 A1**

## Description

### Technical Field

The present invention relates to the field of pharmaceuticals and health foods, and specifically to a carob polysaccharide for stopping cough, reducing phlegm, and enhancing immunity, as well as a method for preparing and using the same.

### Background Art

Carob is distributed in various regions including the Mediterranean coast, the state of California in the United States, and Australia, and is mainly cultivated in regions such as Guangxi, Sichuan, Yunnan, and Guangdong in China. Carob is low in calories, contains no fat, and contains abundant natural polysaccharides, multiple types of vitamins, proteins, and trace elements. At present, carob extract is mainly used in the fields of food flavoring, natural food thickeners, and stabilizers. Carob not only has very high nutritional value, but also exhibits significant pharmacological effects. Syrup made from carob pods can exert a good soothing effect on colds, coughs, and sore throats. The main component of carob pods is sugar.

Respiratory diseases are among the common diseases and are generally accompanied by symptoms such as coughing, expectoration, and even hemoptysis, and often lead to a decline in the body's immunity. Various types of antitussive and expectorant drugs are currently available on the market. Treatment with Western medicine cannot fundamentally cure diseases, tends to result in drug resistance, and is often associated with significant side effects. Traditional Chinese medicine products act slowly and have a strong odor. On the other hand, products with pleasant taste and aroma tend to have limited therapeutic efficacy. Therefore, it is extremely important to develop products that act quickly, have high therapeutic efficacy, and can simultaneously enhance immunity.

### Summary of the Invention

### Problem to be Solved by the Invention

Embodiments of the present invention provide a carob polysaccharide that can improve the airways, stop coughing, reduce phlegm, and further enhance immunity. The carob polysaccharide according to embodiments of the present invention has a wide range of applications, a short treatment period, rapid onset of efficacy, and no toxicity or side effects.

### Means for Solving the Problem

According to one aspect of the present invention, there is provided a carob polysaccharide that is composed of two types of monosaccharides, galactose and glucose, wherein the molar ratio of galactose to glucose is (88-92):(0.8-1.2), and the monosaccharides are linked and combined through glycosidic bonds of (1→4)-Glu, (1→3,4,6)-Glu, (1→3)-Gal, (3→6)-Gal, (2→4,6)-Gal, (2→6)-Gal, and (1→)-Gal.

In some embodiments of the present invention, in the carob polysaccharide, the molar ratio of galactose to glucose is 89.666:1.068.

In the present specification, Glu refers to glucose, and Gal refers to galactose.

In some embodiments of the present invention, the molecular weight of the carob polysaccharide is $(1\text{-}6) \times 10^6$, and may also be $(4.8\text{-}5) \times 10^6$.

In some embodiments of the present invention, the molecular weight of the carob polysaccharide is $(1\text{-}2) \times 10^6$.

In some embodiments of the present invention, the molecular weight of the carob polysaccharide is $(2\text{-}3) \times 10^6$.

In some embodiments of the present invention, the molecular weight of the carob polysaccharide is $(3\text{-}4) \times 10^6$.

In some embodiments of the present invention, the average molecular weight of the carob polysaccharide is $(4.8\text{-}5) \times 10^6$.

In some embodiments of the present invention, the molecular weight distribution width (Mw/Mn) of the carob polysaccharide is 1.5-1.6, and may also be 1.574.

In some embodiments of the present invention, the carob polysaccharide is isolated from carob pods.

In some embodiments of the present invention, the carob polysaccharide has at least the effects of stopping cough, reducing phlegm, and enhancing immunity.

According to another aspect of the present invention, the use of the carob polysaccharide described herein as a pharmaceutical or health product is provided.

According to another aspect of the present invention, the use of the carob polysaccharide described herein in the preparation of a pharmaceutical for stopping cough and reducing phlegm is provided.

According to another aspect of the present invention, the use of the carob polysaccharide described herein in the preparation of a pharmaceutical for enhancing immunity is provided.

According to another aspect of the present invention, there is further provided an effective amount of the above-mentioned carob polysaccharide for treating a disease.

Here, the disease includes coughing, expectoration, and decreased immunity.

According to another aspect of the present invention, there is provided a pharmaceutical composition comprising the carob polysaccharide described herein and a pharmaceutically acceptable carrier.

According to another aspect of the present invention, there is provided a food product comprising the carob polysaccharide described herein, and further comprising a primary food ingredient and a secondary food ingredient.

In some embodiments of the present invention, the food product includes functional foods and dietary supplements.

In some embodiments of the present invention, the food product may be one or more selected from among beverages, candies, syrups, sweeteners, and flavorings.

According to another aspect of the present invention, there is further provided a method for treating a disease, comprising administering to a subject or patient in need thereof, via an oral or non-oral route, an effective amount of the above-mentioned carob polysaccharide, the pharmaceutical composition, or the food product. Here, the disease includes coughing, expectoration, and decreased immunity.

According to another aspect of the present invention, there is further provided a method for preparing the carob polysaccharide, wherein the carob polysaccharide described in the present invention can be prepared by the method, and such that the purity can reach 93% or more.

A method for preparing a carob polysaccharide, comprising: taking pulverized seedless carob pods; performing defatting using 90-95% ethanol in an amount of 1-5 times the weight of the pods; drying the defatted fine powder; adding water and performing extraction; centrifuging the extract and concentrating the supernatant; adding ethanol to the concentrated supernatant to adjust the ethanol mass fraction to 55-60%, centrifuging, and collecting the precipitate; dispersing the obtained precipitate in water; removing proteins by the Sevag method; performing adsorption using macroporous adsorption resin D101; eluting with distilled water to collect the mobile phase components; performing DEAE-52 cellulose column chromatography and eluting with distilled water; performing Sephadex G-100 gel filtration chromatography and collecting the eluate using deionized water as the mobile phase; further performing Sephadex G-150 gel filtration chromatography and collecting the eluate using deionized water as the mobile phase; combining the eluates with the same elution peak; concentrating under reduced pressure; and freeze-drying to obtain the carob polysaccharide.

In some embodiments of the present invention, carob pods are pulverized into fine powder of 80-300 mesh to destroy the cell structure of the pods, which, while being advantageous for the elution of intracellular substances, also increases the contact area between the pod powder and the extraction solvent, thereby improving the extraction rate. In some embodiments of the present invention, ultrafine pulverization technology may be used for pulverization.

In some embodiments of the present invention, the weight of ethanol used for defatting is 2-5 times the weight of the seedless carob pods.

In some embodiments of the present invention, the extraction temperature is 40-50°C, for example, 45°C. Within this temperature range, it is advantageous in sufficiently eluting the carob polysaccharide.

In some embodiments of the present invention, extraction is performed 2-4 times, with each extraction lasting 1-5 hours.

In some embodiments of the present invention, the filtrate (extract) is subjected to ultracentrifugation at 5,000 rpm for 10 minutes, and the supernatant is combined.

In some embodiments of the present invention, the supernatant is concentrated under reduced pressure at 60°C and 100 Pa until it becomes a viscous and concentrated solution.

In some embodiments of the present invention, the carob pods are dried pods of *Ceratonia siliqua Linn.,* a plant of the genus *Ceratonia* in the family *Fabaceae.*

In an experiment, mice were placed in a large beaker containing concentrated ammonia water to induce coughing, and the number of coughs was measured for the groups receiving forced oral administration of different concentrations of the carob polysaccharide Hocacy, a positive control group, and a blank control group. In addition, the secretion of phenol red in the trachea of the mice was analyzed, and it was shown that the carob polysaccharide Hocacy of the embodiments of the present invention has a good antitussive and expectorant effect. In an experiment in which the improvement effect on the reduction of T cells in genetically modified zebrafish emitting red fluorescence was examined, it was demonstrated that the carob polysaccharide Hocacy of the embodiments of the present invention has an effect of enhancing immunity.

## Brief Description of the Drawings

FIG. 1 is a chromatogram showing the molecular weight of the carob polysaccharide Hocacy of the embodiment of the present invention.

FIG. 2 is an infrared absorption spectrum of the carob polysaccharide Hocacy of the embodiment of the present invention.

FIG. 3 is a total ion chromatogram by GC-MS of the carob polysaccharide Hocacy of the embodiment of the present invention.

FIG. 4 is an HPLC chromatogram of the carob polysaccharide Hocacy.

FIG. 5 is an identification spectrum of the fragment ion of galactose as a monosaccharide component of the carob polysaccharide Hocacy.

FIG. 6 is an identification spectrum of the fragment ion of galactose as a monosaccharide component of the carob polysaccharide Hocacy.

FIG. 7 is an identification spectrum of the fragment ion of galactose as a monosaccharide component of the carob polysaccharide Hocacy.

FIG. 8 is an identification spectrum of the fragment ion of glucose as a monosaccharide component of the carob polysaccharide Hocacy.

FIG. 9 is an identification spectrum of the fragment ion of galactose as a monosaccharide component of the carob polysaccharide Hocacy.

FIG. 10 is an identification spectrum of the fragment ion of glucose as a monosaccharide component of the carob polysaccharide Hocacy.

FIG. 11 is an identification spectrum of the fragment ion of glucose as a monosaccharide component of the carob polysaccharide Hocacy.

FIG. 12 is an infrared absorption spectrum after methylation of the carob polysaccharide Hocacy of the embodiment of the present invention.

FIG. 13 is a schematic diagram showing the effect of the carob polysaccharide Hocacy on the latent period of cough in mice.

FIG. 14 is a schematic diagram showing the effect of the carob polysaccharide Hocacy on the number of coughs within two minutes in mice.

FIG. 15 is a schematic diagram showing the effect of the carob polysaccharide Hocacy on the amount of phenol red secretion in mice.

FIG. 16 is a schematic diagram showing the effect of the carob polysaccharide Hocacy on daily body weight changes in mice.

FIG. 17 is a comparative diagram of the fluorescence intensity of T cells in zebrafish between the carob polysaccharide Hocacy group and the model control group.

## Mode for Carrying Out the Invention

Hereinafter, the invention will be further described in conjunction with examples; however, the scope of the present invention is not limited thereto.

The carob pods described below are dried pods of *Ceratonia siliqua Linn., a* plant of the genus *Ceratonia* in the family *Fabaceae,* and were provided by SHENZHEN AIMIN MEDICINE TECHNOLOGY CO., LTD.

The macroporous adsorption resin D101, Sephadex G-150, Sephadex G-100, and DEAE-52 cellulose used below were all commercially purchased products.

## Example 1: Extraction and Isolation of Carob Polysaccharide Hocacy

8 kg of seedless carob pods were pulverized and defatted using 95% ethanol at three times the weight of the pods, followed by drying. Deionized water was added at a solid-to-liquid ratio of 1:2.3 (g/mL), and extraction was performed four times, each for 2 hours, at an extraction temperature of 45°C. The extracts were combined. The combined extract was centrifuged (5,000 rpm, 10 min), and the supernatant was concentrated under reduced pressure at 60°C and 100 Pa until it became viscous. Ethanol was slowly added to the resulting concentrate to adjust the ethanol mass fraction to 60%, followed by centrifugation to collect the precipitate, thereby obtaining crude polysaccharides. The crude polysaccharides were dispersed in purified water at 20 times the weight and subjected to protein removal using the Sevag method, which was repeated six times. The polysaccharides after protein removal were dispersed in water at 10 times the weight, subjected to adsorption using macroporous adsorption resin D101, and eluted with distilled water at twice the column volume to collect the mobile phase component. DEAE-52 cellulose column chromatography was performed, and elution was carried out with distilled water at twice the column volume. Subsequently, Sephadex G-100 gel filtration chromatography and Sephadex G-150 gel filtration chromatography were performed in sequence, and the eluates were collected using deionized water as the mobile phase. The eluates with the same elution peak were combined, concentrated under reduced pressure, and freeze-dried to obtain carob polysaccharide Hocacy (polysaccharide content: 93%).

The method for measuring the content of the carob polysaccharide Hocacy in this example is as follows.

Precisely 9.6 mg of the carob polysaccharide Hocacy was weighed and dissolved in purified water to prepare a solution with a concentration of 0.25 mg/mL. For 1.0 mL of the test solution, the mass concentration of the polysaccharide in the solution was measured using the phenol-sulfuric acid method, based on the standard curve of glucose and the regression equation.

The standard curve of glucose is $y = 1.3421x + 0.2255$ ($R^2 = 0.9992$).

Here, $x$ represents the concentration of the polysaccharide (mg/mL), and y represents the absorbance.

Polysaccharide content (%) = (mass concentration ($\mu$g/mL) $\times$ dilution factor $\times$ volume (mL)) / (dry weight of polysaccharide (g) $\times$ 1000)

**Experimental Example 1**

**Structural Analysis of Carob Polysaccharide Hocacy (prepared in Example 1)**

(1) Molecular Weight Measurement of Carob Polysaccharide Hocacy

Precisely 10.0 mg of the carob polysaccharide Hocacy was weighed, and a solution with a concentration of 2 mg/mL was prepared. Detection was performed using gel permeation chromatography (GPC), a differential refractive index detector, and a light scattering detector. The mobile phase was a 0.1 mol/L $NaNO_3$ solution, and the flow rate was set to 0.7 mL/min.

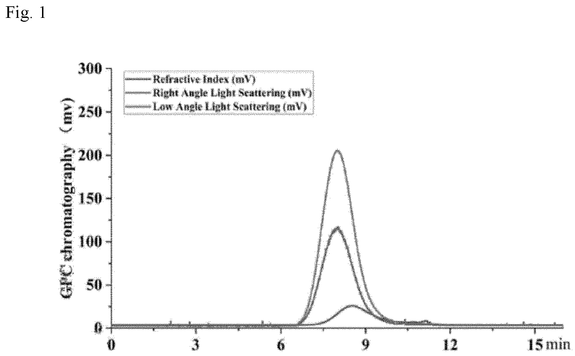

The molecular weight measurement result of the carob polysaccharide Hocacy is shown in FIG. 1 (GPC). Based on analysis and calculation, the average molecular weight was $4.895 \times 10^6$, and the molecular weight distribution width (Mw/Mn) was 1.574, indicating a narrow distribution and proving the high purity of the carob polysaccharide Hocacy. In FIG. 1, the horizontal axis represents retention time and the vertical axis represents refractive index. "GPC chromatography" refers to gel permeation chromatography, "Refractive Index (mV)" refers to the refractive index, "Right Angle Light Scattering (mV)" refers to right-angle light scattering, and "Low Angle Light Scattering (mV)" refers to low-angle light scattering.

(2) Infrared Absorption Spectrum (IR) Analysis of Carob Polysaccharide Hocacy

5 mg of the carob polysaccharide Hocacy sample was weighed, and an appropriate amount of dry KBr was added and ground to prepare a uniform and transparent tablet. The analysis was conducted using a Fourier transform infrared spectrophotometer in the range of 4000 cm$^{-1}$ to 400 cm$^{-1}$.

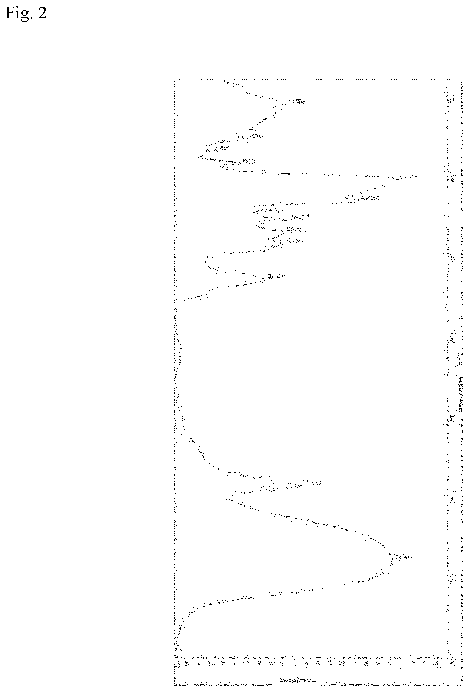

The infrared absorption spectrum of the carob polysaccharide Hocacy is shown in FIG. 2. According to FIG. 2, an absorption peak due to the stretching vibration of - OH appears at 3385 cm$^{-1}$, an absorption peak due to the stretching vibration of -CH$_2$ appears at 2927 cm$^{-1}$, and a strong absorption peak appears at 1020 cm$^{-1}$, corresponding to the stretching vibration of C-O. The vertical axis of FIG. 2 indicates transmittance (%).

(3) Analysis of Monosaccharide Composition of Carob Polysaccharide Hocacy

A 5.5 mg sample of carob polysaccharide Hocacy was weighed, and 1 mL of 2 mol/L trifluoroacetic acid (TFA) was added. The mixture was sealed under nitrogen gas and heated at 110°C for 120 minutes to perform hydrolysis. After dissolving the hydrolyzed sample, 90 mg of NaBH$_4$ was added and a reduction reaction was carried out for 4 hours. The reduced sample was dried in an oven, followed by the addition of 5 mL of acetic anhydride, and acetylation was carried out at 110°C. The final acetylated monosaccharides were dissolved in ethyl acetate, filtered through a membrane, and structurally identified using GC-MS.

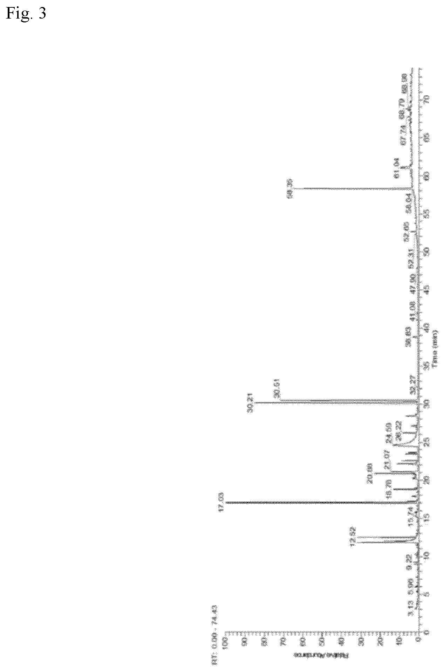

After hydrolysis and acetylation of the carob polysaccharide Hocacy, structural identification was performed by GC-MS. The total ion chromatogram is shown in FIG. 3. In FIG. 3, the horizontal axis labeled "Time" indicates the peak detection time, and the vertical axis labeled "Relative abundance" indicates relative intensity.

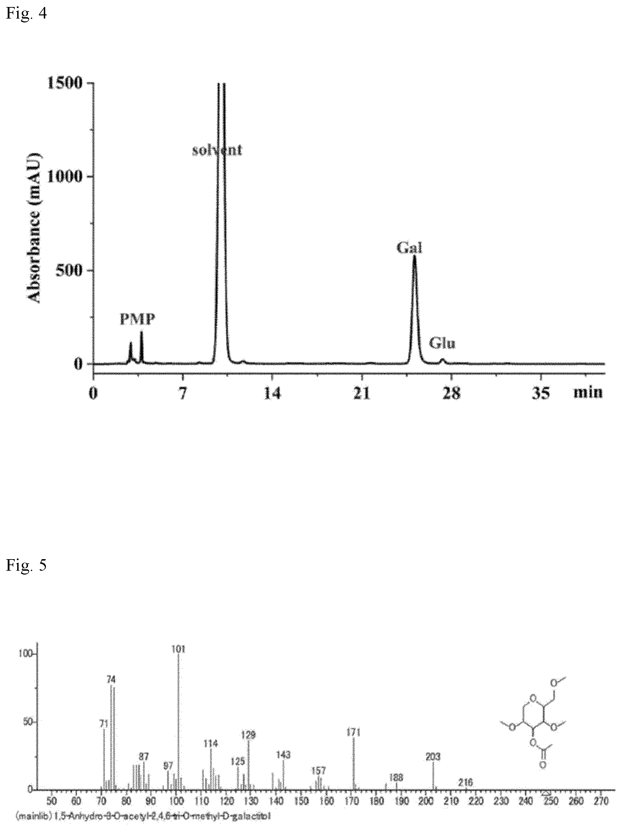

According to HPLC and spectral library analysis, the monosaccharide composition (molar ratio) of the carob polysaccharide Hocacy is galactose:glucose = 89.666:1.068, and the presence of fructose in the carob polysaccharide Hocacy was not detected by the HPLC-ELSD method. The HPLC chromatogram is as shown in FIG. 4. In FIG. 4, the horizontal axis represents time, the vertical axis labeled "Absorbance (mAU)" indicates absorbance, "solvent" represents the solvent peak, "PMP" denotes the derivatization reagent (1-phenyl-3-methyl-5-pyrazolone), "Gal" indicates galactose, and "Glu" indicates glucose.

Based on spectral library analysis, the following conclusions were obtained.

The analysis results of the monosaccharide composition of the carob polysaccharide Hocacy are shown in FIGS. 5 to 11.

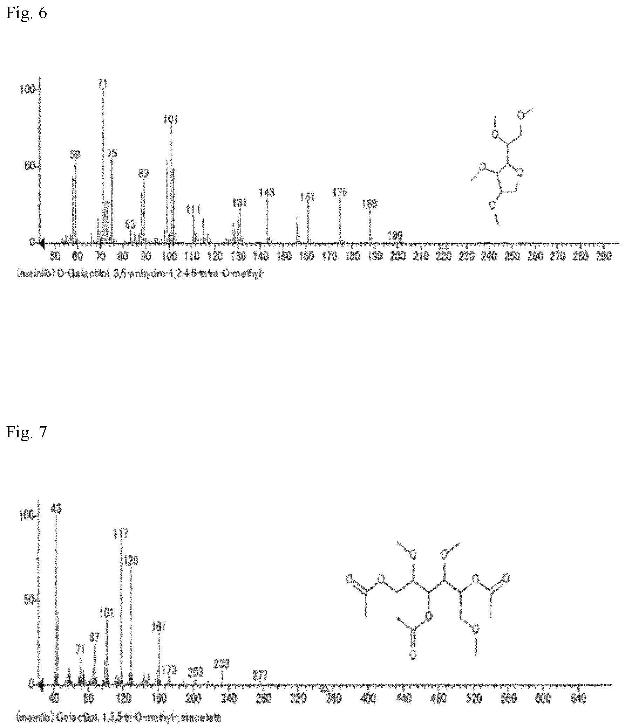

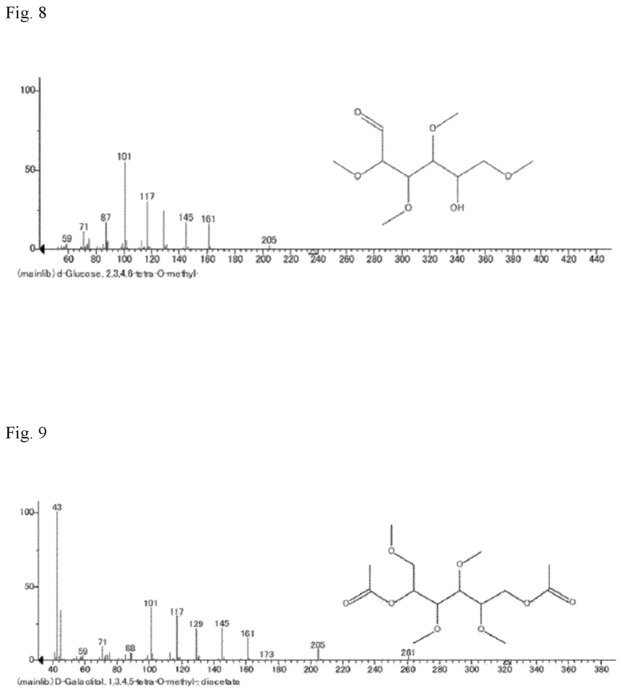

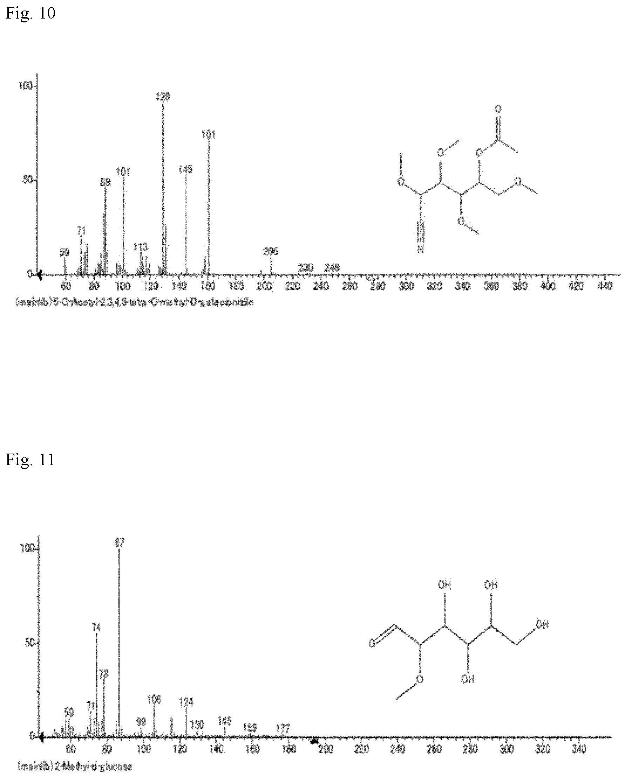

FIG. 5 is an identification spectrum of the fragment ion of galactose as a monosaccharide component of the carob polysaccharide Hocacy, where t = 17.03. FIG. 6 is an identification spectrum of the fragment ion of galactose, where t = 18.78. FIG. 7 is an identification spectrum of the fragment ion of galactose, where t = 20.88. FIG. 8 is an identification spectrum of the fragment ion of glucose, where t = 30.21. FIG. 9 is an identification spectrum of the fragment ion of galactose, where t = 30.51. FIG. 10 is an identification spectrum of the fragment ion of glucose, where t = 38.83. FIG. 11 is an identification spectrum of the fragment ion of glucose (2-methylglucose), where t = 61.04.

In FIG. 5, (mainlib) 1,5-Anhydro-3-O-acetyl-2,4,6-tri-O-methyl-D-galactitol indicates (main library) 1,5-anhydro-3-O-acetyl-2,4,6-tri-O-methyl-D-galactose. In FIG. 6, (mainlib) D-Galactitol, 3,6-anhydro-1,2,4,5-tetra-O-methyl- indicates (main library) 3,6-anhydro-1,2,4,5-tetra-O-methyl-D-galactose. In FIG. 7, (mainlib) Galactitol, 1,3,5-tri-O-methyl-, triacetate indicates (main library) 1,3,5-tri-O-methyl-galactose, triacetate. In FIG. 8, (mainlib) D-Glucose, 2,3,4,6-tetra-O-methyl- indicates (main library) 2,3,4,6-tetra-O-methyl-D-glucose. In FIG. 9, (mainlib) D-Galactitol, 1,3,4,5-tetra-O-methyl-, diacetate indicates (main library) 1,3,4,5-tetra-O-methyl-D-galactose, diacetate. In FIG. 10, (mainlib) 5-O-Acetyl-2,3,4,6-tetra-O-methyl-galactonitrile indicates (main library) 2,3,4,6-tetra-O-methyl-galactonitrile. In FIG. 11, (mainlib) 2-methyl-D-glucose indicates (main library) 2-methyl-D-glucose.

(4) Measurement of Monosaccharide Linkage Positions in Carob Polysaccharide Hocacy

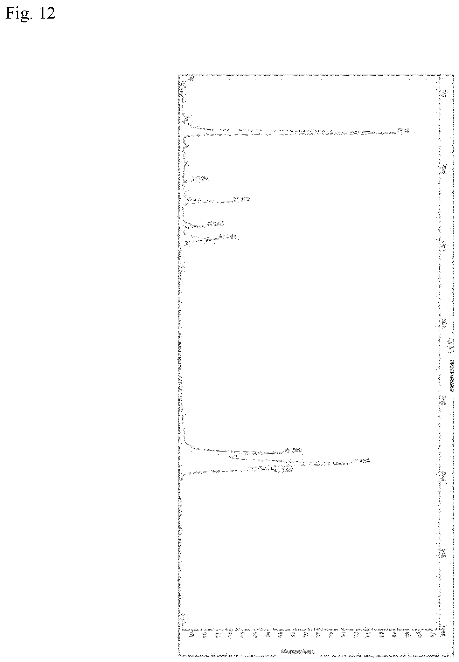

A 20 mg sample of the carob polysaccharide Hocacy was weighed and dissolved in 6 mL of dimethyl sulfoxide (DMSO). The solution was magnetically stirred at 40°C for 15 hours until the polysaccharide sample was completely dissolved. After methylation, hydrolysis, and acetylation of the carob polysaccharide Hocacy, a total ion chromatogram was obtained by GC-MS, as shown in FIG. 3. In FIG. 3, the horizontal axis labeled "Time" represents the peak detection time, and the vertical axis labeled "Relative abundance" represents relative intensity. The infrared absorption spectrum of the carob polysaccharide Hocacy after methylation is shown in FIG. 12.

Analysis of the fragment ions in the mass spectrum revealed that the carob polysaccharide Hocacy possesses the following glycosidic linkages: (1→4)-Glu, (1→3,4,6)-Glu, (1→3)-Gal, (3→6)-Gal, (2→4,6)-Gal, (2→6)-Gal, and (1→)-Gal. The analysis results are shown in Table 1. Here, Glu refers to glucose, and Gal refers to galactose.

Table 1. Methylation Analysis of Carob Polysaccharide Hocacy

| Monosaccharide | Methylated Product | Linkage Type |
|---|---|---|
| Galactose | 2,4,6-Me$_3$-Gal | 1,3-linked Gal |
| | 1,2,4-Me$_3$-Gal | 3,6-linked Gal |
| | 1,3-Me$_2$-Gal | 2,4,6-linked Gal |
| | 1,3,4-Me$_3$-Gal | 2,6-linked Gal |
| | 2,3,4,6-Me$_4$-Gal | 1-linked Gal |
| Glucose | 2,3,6-Me$_3$-Glu | 1,4-linked Glu |
| | 2-Me-Glu | 1,3,4,6-linked Glu |

Experimental Example 2

Experiment on the effect of the carob polysaccharide Hocacy (prepared in Example 1) on airway function in mice whose coughing was induced and stimulated by ammonia water

Mice were placed in a large beaker containing concentrated ammonia water to induce coughing.

1.Effect of drug administration on the number of coughs in mice

The experiment was divided into five groups, each set as follows. That is, Group 1 was set as the blank group to which physiological saline was administered; Group 2 was set as the positive group to which benproperine phosphate (Tonghua Changcheng Pharmaceutical Co., Ltd., approval number: H22020149) was administered at 60 mg/kg; and Groups 3 to 5 were set as the treatment groups to which the carob polysaccharide Hocacy was administered at low dose (100 mg/kg), medium dose (200 mg/kg), and high dose (400 mg/kg), respectively. Forced oral administration was carried out for 14 days. One hour after the final forced oral administration was completed, a 1000 mL large beaker was inverted and placed on the laboratory bench. Then, 25% concentrated ammonia water was continuously sprayed into the beaker for 10 seconds using a sprayer. The mice were placed into the beaker, and time measurement was started. The indicators were contraction of abdominal muscles, breathing with a widely opened mouth, and sounds of coughing. The time until the mouse first coughed in the beaker (latent period) and the number of coughs within two minutes were recorded. The results are shown in Table 2.

According to the results, after stimulation with ammonia water to induce coughing, the degree of coughing differed among the groups. All of the high-, medium-, and low-dose groups of the carob polysaccharide Hocacy significantly reduced the number of coughs in mice induced by ammonia water and prolonged the latent period. Compared with the blank group, the treatment groups showed significant or very significant effects (****$p < 0.0001$, ***$p < 0.001$, **$p < 0.05$), indicating that the carob polysaccharide Hocacy has a certain degree of antitussive effect against ammonia-induced coughing in mice and was shown to exhibit dose dependency. The antitussive effect of the high-dose group (400 mg/kg) was the most pronounced, and the time to first cough in both the high-dose and medium-dose groups was significantly prolonged compared with the positive group ($p < 0.01$, $p < 0.05$).

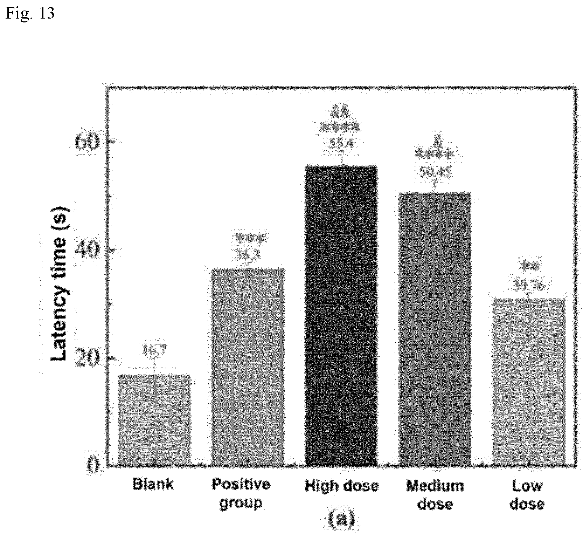

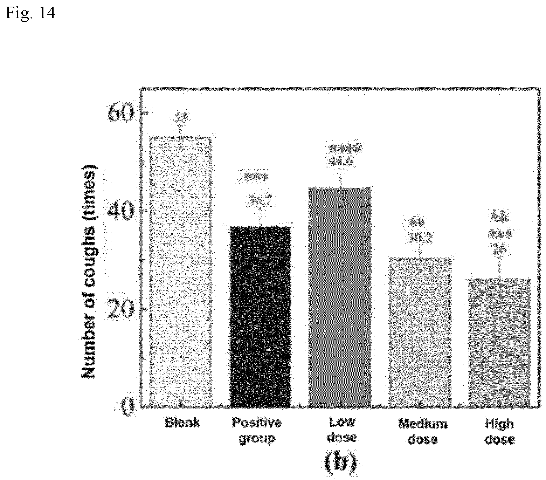

The number of coughs during two minutes in the high-dose group is significantly different from that in the positive group ($p < 0.01$). Accordingly, the carob polysaccharide Hocacy possesses a strong antitussive effect. A schematic diagram representing the effect of the carob polysaccharide Hocacy on the latent period of coughing in mice is as shown in FIG. 13, and a schematic diagram representing the number of coughs within two minutes is as shown in FIG. 14.

Table 2

| Group | Dosage | Time to first cough (sec) | Number of coughs within 2 min (times) |
|---|---|---|---|
| Blank group | Physiological saline | 16.70 ± 3.33 | 47.9 ± 4.31 |
| Positive group (benproperine phosphate) | 60 mg/kg (forced oral administration) | 36.30 ± 1.27*** | 28.5 ± 4.79*** |
| High-dose group | 400 mg/kg (forced oral administration) | 55.40 ± 2.88****&& | 14.6 ± 3.74****&& |
| Medium-dose group | 200 mg/kg (forced oral administration) | 50.45 ± 2.51****& | 22.3 ± 1.89*** |
| Low-dose group | 100 mg/kg (forced oral administration) | 30.76 ± 1.24** | 39.3 ± 4.01** |
| **Note:** Compared to blank group: ****$p < 0.0001$, ***$p < 0.001$, **$p < 0.01$<br>Compared to positive group: &&$p < 0.01$, &$p < 0.05$ | | | |

## 2. Effect of drug administration on expectoration in mice

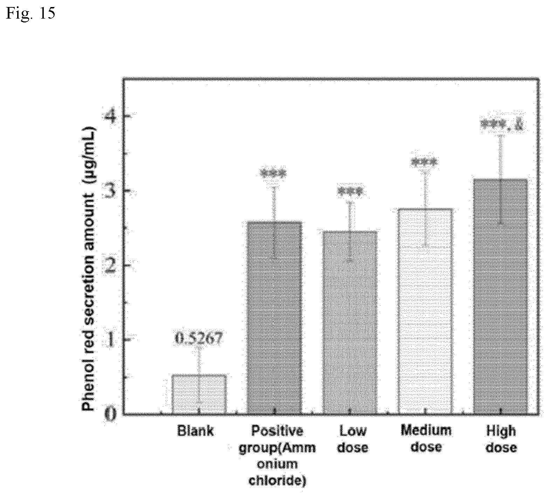

Each group of mice was continuously administered for another two days, and on the 16th day, 30 minutes after the final administration, 0.5 mL per mouse of 5% phenol red physiological saline was intraperitoneally injected, and 30 minutes after the injection, the mice were euthanized using the cervical dislocation method. The trachea from the thyroid cartilage to the bronchi was excised, and in order to completely release the phenol red inside the trachea, ultrasonic washing was performed for 10 minutes using 3 mL of physiological saline and 0.1 mL of 1 mol/L sodium hydroxide solution. The absorbance of the solution was measured at 546 nm, and the content of phenol red was calculated according to the regression equation of the standard curve, and the result is as shown in Table 3, which shows that the carob polysaccharide Hocacy, in all cases, significantly increased the secretion amount of phenol red in the trachea of mice, and that in all cases, there was a significant difference compared with physiological saline (***$p < 0.001$), and that the carob polysaccharide Hocacy has a certain effect of reducing phlegm. In the case of the high dose, there was a significant difference compared with the positive control group ($p < 0.05$), and dose dependency was exhibited. A schematic diagram showing the effect of the carob polysaccharide Hocacy on the secretion amount of phenol red in mice is as shown in FIG. 15.

**Table 3.**

| Group | Dosage | Phenol red secretion amount ($\mu$g/mL) |
|---|---|---|
| Blank group | Physiological saline | 0.5267 $\pm$ 0.6671 |
| Positive group (Ammonium chloride) | 500 mg/kg (forced oral administration) | 2.5741 $\pm$ 0.7873*** |
| High-dose group | 400 mg/kg (forced oral administration) | 3.1510 $\pm$ 0.8456****& |
| Medium-dose group | 200 mg/kg (forced oral administration) | 2.7543 $\pm$ 0.0944*** |
| Low-dose group | 100 mg/kg (forced oral administration) | 2.4449 $\pm$ 1.022*** |
| **Note:** Compared to blank group: ***p < 0.001; Compared to positive group: &p < 0.05 | | |

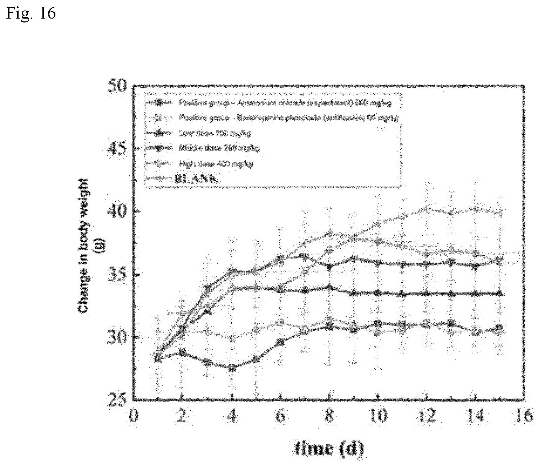

In addition, upon tracking the daily body weight changes in mice, it was found that the mice administered the carob polysaccharide Hocacy (at high, medium, and low doses) all showed less weight loss than the positive groups (ammonium chloride and benproperine phosphate), and exhibited a tendency of steady weight gain, second only to the blank group. In contrast, the body weight of the mice in the two positive groups showed almost no change, and there was a marked difference compared with the polysaccharide groups, and it was found that their body weight was lower. It was thereby demonstrated that the polysaccharide from carob pods has no toxicity or side effects and possesses good suitability. The changes in body weight are as shown in FIG. 16.

As described above, through the present study, it was demonstrated that the carob polysaccharide Hocacy possesses significant antitussive and expectorant effects, and that it has no notable toxicity or side effects, and the present study establishes a certain basis for the development of a new drug using the polysaccharide from carob pods.

**Experimental Example 3**

**Improvement effect of the carob polysaccharide Hocacy (prepared in Example 1) on the reduction of T cells in zebrafish**

The zebrafish, in which T cells were genetically modified to emit red fluorescence, were bred through natural mating. All zebrafish at 3 days post-fertilization (3 dpf) were raised at 28°C in aquaculture water (water quality: reverse osmosis water per 1 L supplemented with 200 mg of rapidly dissolving sea salt, having a conductivity of 450-550 $\mu$S/cm, a pH of 6.5-8.5, and a hardness of 50-100 mg/L CaCO$_3$), and the zebrafish were bred and provided by the Fish Breeding Center of Beijing HuanTe ZhiYu Youjian Biotechnology Co., Ltd.

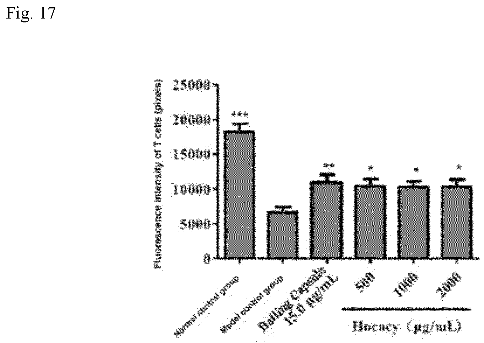

Zebrafish at 3 dpf, in which T cells were genetically modified to emit red fluorescence, were randomly selected into six-well plates, and 30 zebrafish were treated per well (experimental groups). When administered by dissolving in water, Hocacy at 20.0 mg/mL was prepared in standard dilution water (the concentrations are shown in Table 4), and the positive control was set as Bailing Capsule at a concentration of 15.0 $\mu$g/mL (Hangzhou Zhongmei Huadong Pharmaceutical Co., Ltd., approval number: 2111064), and a normal control group and a model control group were established simultaneously, with the volume of each well set to 3 mL. In order to construct a zebrafish immunodeficiency model, vinorelbine tartrate injection was intravenously administered to each experimental group except the normal control group. The treatment was continued at 28°C until 5 dpf, and 10 zebrafish were randomly selected from each experimental group, and they were photographed under a fluorescence microscope, and data were analyzed and collected using NIS-Elements D3.20 advanced image processing software, and the fluorescence intensity of T cells in zebrafish was analyzed, and based on the statistical analysis results of this indicator, the improvement effect of Hocacy on the reduction of T cells was evaluated. The results of statistical processing are expressed as mean $\pm$ SE. Statistical analysis was performed using SPSS 26.0 software, and p < 0.05 indicates a statistically significant difference. The experimental results on the improvement effect of Hocacy on the reduction of T cells are as shown in Table 5, and the comparison of the fluorescence intensity of T cells between the zebrafish treated with Hocacy and the model control group is as shown in FIG. 17, and the results show that Hocacy has a good improvement effect on the reduction of T cells, and it does not exhibit a clear dose dependency. Therefore, the carob polysaccharide Hocacy has an effect of enhancing immunity.

**Table 4. Results of the Study on the Effect of Hocacy Concentration in Improving T Cell Reduction (n = 30)**

| Group | Concentration ($\mu$g/mL) | Number of deaths (n) | Mortality rate (%) | Phenotype |
|---|---|---|---|---|
| Normal control group | - | 0 | 0 | No obvious abnormalities observed |

(continued)

| Group | Concentration (μg/mL) | Number of deaths (n) | Mortality rate (%) | Phenotype |
|---|---|---|---|---|
| Model control group | - | 0 | 0 | No obvious abnormalities observed |
| Positive control | 15 | 0 | 0 | No obvious abnormalities observed |
| Hocacy | 125 | 0 | 0 | Similar to model control group |
| Hocacy | 250 | 0 | 0 | Similar to model control group |
| Hocacy | 500 | 0 | 0 | Similar to model control group |
| Hocacy | 1000 | 0 | 0 | Similar to model control group |
| Hocacy | 2000 | 0 | 0 | Similar to model control group |

**Table 5. Experimental Results of the Improvement Effect of Hocacy on T Cell Reduction (n = 10)**

| Group | Concentration (μg/mL) | T cell fluorescence intensity (pixels, mean $\pm$ SE) |
|---|---|---|
| Normal control group | - | 18221 $\pm$ 1183*** |
| Model control group | - | 6657 $\pm$ 764 |
| Bailing Capsule | 15.0 | 10983 $\pm$ 1124** |
| Hocacy | 500 | 10399 $\pm$ 1071* |
| Hocacy | 1000 | 10308 $\pm$ 837* |
| Hocacy | 2000 | 10338 $\pm$ 1078* |
| **Note:** Compared to model control group: *$p < 0.05$, **$p < 0.01$, ***$p < 0.001$ | | |

It should be noted that, in the above, the present invention has been described in detail through general explanation and specific embodiments, but it is self-evident to those skilled in the art that some modifications and improvements can be made based on the present invention. Therefore, all such modifications and improvements that are made without departing from the spirit of the present invention belong to the scope intended to be protected by the present invention.

## Claims

1. A carob polysaccharide, comprising two types of monosaccharides, galactose and glucose, wherein the molar ratio of galactose to glucose is (88-92):(0.8-1.2), and the monosaccharides are linked and combined through glycosidic bonds of $(1{\rightarrow}4)$-Glu, $(1{\rightarrow}3,4,6)$-Glu, $(1{\rightarrow}3)$-Gal, $(3{\rightarrow}6)$-Gal, $(2{\rightarrow}4,6)$-Gal, $(2{\rightarrow}6)$-Gal, and $(1{\rightarrow})$-Gal.

2. The carob polysaccharide according to claim 1, wherein the molar ratio of galactose to glucose is 89.666:1.068.

3. The carob polysaccharide according to claim 1 or 2, wherein the molecular weight of the carob polysaccharide is $(1\text{-}6){\times}10^6$, optionally $(4.8\text{-}5){\times}10^6$, and further optionally $4.895{\times}10^6$; and/or wherein the molecular weight distribution (Mw/Mn) of the carob polysaccharide is 1.5-1.6, optionally 1.574.

4. A method for preparing a carob polysaccharide, comprising:

(i) taking pulverized seedless carob pods, performing defatting using 90-95% ethanol, and drying the defatted fine powder;
(ii) adding water and performing extraction, centrifuging the extract, concentrating the supernatant, adding ethanol to adjust the ethanol mass fraction to 55-60%, centrifuging, and collecting the precipitate;
(iii) dispersing the obtained precipitate in water, removing proteins by the Sevag method, performing adsorption using macroporous adsorption resin D101, eluting with distilled water to collect the mobile phase components, performing DEAE-52 cellulose column chromatography, eluting with distilled water, performing Sephadex G-100 gel filtration chromatography, collecting the eluate using deionized water as the mobile phase, further performing Sephadex G-150 gel filtration chromatography, collecting the eluate using deionized water as the mobile phase, combining the fractions with the same elution peak, concentrating under reduced pressure, and freeze-drying to obtain the carob polysaccharide.

5. The method for preparing a carob polysaccharide according to claim 4,
wherein the carob pods are pulverized to 80-300 mesh; and/or the solid-liquid ratio during extraction is 1:(2-5) in g/mL; and/or the extraction temperature is 40-50°C.

6. A carob polysaccharide prepared by the method according to claim 4 or 5.

7. Use of the carob polysaccharide according to any one of claims 1 to 3 or 6 as a pharmaceutical, optionally for use in the preparation of a pharmaceutical for stopping cough and reducing phlegm and/or for enhancing immunity.

8. A pharmaceutical composition comprising the carob polysaccharide according to any one of claims 1 to 3 or 6, and further comprising a pharmaceutically acceptable carrier.

9. A food comprising the carob polysaccharide according to any one of claims 1 to 3 or 6, and further comprising a primary food ingredient and a secondary food ingredient.

10. A method for treating a disease, comprising administering, via an oral or non-oral route, to a subject or patient in need thereof an effective amount of the carob polysaccharide according to any one of claims 1 to 3 or 6, the pharmaceutical composition according to claim 8, or the food according to claim 9, wherein the disease may comprise cough, sputum production, or reduced immunity.

Fig. 1

Fig. 2

Fig. 3

Fig. 4

Fig. 5

(mainlib) 1,5-Anhydro-3-O-acetyl-2,4,6-tri-O-methyl-D-galactitol

Fig. 6

(mainlib) D-Galactitol, 3,6-anhydro-1,2,4,5-tetra-O-methyl-

Fig. 7

(mainlib) Galactitol, 1,3,5-tri-O-methyl-, triacetate

Fig. 8

(mainlib) d-Glucose, 2,3,4,6-tetra-O-methyl-

Fig. 9

(mainlib) D-Galactitol, 1,3,4,5-tetra-O-methyl-, diacetate

Fig. 10

(mainlib) 5-O-Acetyl-2,3,4,6-tetra-O-methyl-D-galactonitrile

Fig. 11

(mainlib) 2-Methyl-d-glucose

Fig. 12

Fig. 13

(a)

Fig. 14

(b)

Fig. 15

Fig. 16

Fig. 17

## INTERNATIONAL SEARCH REPORT

| International application No. |
|---|
| **PCT/JP2024/010763** |

| A. | CLASSIFICATION OF SUBJECT MATTER |
|---|---|

*C08B 37/00*(2006.01)i; *A23L 5/00*(2016.01)i; *A23L 29/00*(2016.01)i; *A23L 33/105*(2016.01)i; *A23L 33/125*(2016.01)i; *A61K 31/715*(2006.01)i; *A61K 36/48*(2006.01)i; *A61P 11/10*(2006.01)i; *A61P 11/14*(2006.01)i; *A61P 37/04*(2006.01)i
FI:   C08B37/00; A23L5/00 N; A23L29/00; A23L33/105; A23L33/125; A61K31/715; A61K36/48; A61P11/10; A61P11/14; A61P37/04

According to International Patent Classification (IPC) or to both national classification and IPC

| B. | FIELDS SEARCHED |
|---|---|

Minimum documentation searched (classification system followed by classification symbols)

C08B37/00; A23L5/00; A23L29/00; A23L33/105; A23L33/125; A61K31/715; A61K36/48; A61P11/10; A61P11/14; A61P37/04

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

JSTPlus/JMEDPlus/JST7580 (JDreamIII)

| C. | DOCUMENTS CONSIDERED TO BE RELEVANT |
|---|---|

| Category* | Citation of document, with indication, where appropriate, of the relevant passages | Relevant to claim No. |
|---|---|---|
| A | JP 10-279486 A (TAIYO KAGAKU CO., LTD.) 20 October 1998 (1998-10-20) claims, examples | 1-10 |
| A | US 2018/0271896 A1 (THE FIRST AFFILIATTED HOSPITAL OF GUANGZHOU MEDICAL UNIVERSITY) 27 September 2018 (2018-09-27) claims, paragraphs [0049]-[0106] | 1-10 |
| A | CN 103356691 A (LANZHOU UNIVERSITY OF TECHNOLOGY) 23 October 2013 (2013-10-23) claims, examples | 1-10 |
| A | RICO, D. et al., In vitro approach for evaluation of carob by-products as source bioactive ingredients with potential to attenuate metabolic syndrome (MetS), Heliyon, 2019, vol. 5, no. 1, e01175, pp. 1-19 abstract | 1-10 |

☑ Further documents are listed in the continuation of Box C.      ☑ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | "&" | document member of the same patent family |
| "P" | document published prior to the international filing date but later than the priority date claimed | | |

| Date of the actual completion of the international search | Date of mailing of the international search report |
|---|---|
| **23 May 2024** | **04 June 2024** |

| Name and mailing address of the ISA/JP | Authorized officer |
|---|---|
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (July 2022)

**EP 4 692 129 A1**

## INTERNATIONAL SEARCH REPORT

International application No.

**PCT/JP2024/010763**

C. DOCUMENTS CONSIDERED TO BE RELEVANT

| Category* | Citation of document, with indication, where appropriate, of the relevant passages | Relevant to claim No. |
|---|---|---|
| A | GOULAS, Vlasios et al., Functional Components of Carob Fruit: Linking the Chemical and Biological Space, International Journal of Molecular Sciences, 2016, vol. 17, no. 11, 1875, pp. 1-20 <br> abstract, 2. Functional Chemical Components of Carob Fruit | 1-10 |

Form PCT/ISA/210 (second sheet) (July 2022)

**INTERNATIONAL SEARCH REPORT**
Information on patent family members

International application No.

**PCT/JP2024/010763**

| Patent document cited in search report | | | Publication date (day/month/year) | Patent family member(s) | | | Publication date (day/month/year) |
|---|---|---|---|---|---|---|---|
| JP | 10-279486 | A | 20 October 1998 | (Family: none) | | | |
| US | 2018/0271896 | A1 | 27 September 2018 | US | 2016/0339054 | A1 | |
| | | | | WO | 2015/144059 | A1 | |
| | | | | EP | 3124032 | A1 | |
| | | | | CN | 104027345 | A | |
| | | | | CN | 104800236 | A | |
| CN | 103356691 | A | 23 October 2013 | (Family: none) | | | |

Form PCT/ISA/210 (patent family annex) (July 2022)